# EUROPEAN PATENT APPLICATION

(11) **EP 4 215 115 A1**
(43) Date of publication of application: **26.07.2023**
(21) Application number: 21866310.2
(22) Date of filing: 28.05.2021
(51) Int. Cl.: A61B 5/16

(54) **SENSIBILITY MEASURING METHOD AND SENSIBILITY MEASURING SYSTEM**

(30) Priority: 10.09.2020 JP 2020152341
(71) Applicant: SHIMADZU CORPORATION, Kyoto-shi, Kyoto 604-8511 (JP)
(72) Inventor: MIZUMOTO, Toru, Kyoto-shi, Kyoto 604-8511 (JP); ICHIOKA, Akina, Kyoto-shi, Kyoto 604-8511 (JP); FURUTA, Masafumi, Kyoto-shi, Kyoto 604-8511 (JP); MURATA, Koichi, Kyoto-shi, Kyoto 604-8511 (JP)
(74) Representative: Kilian Kilian & Partner
(86) International application number: PCT/JP2021/020472
(87) International publication number: WO 2022/054349

(57) **Abstract**

A sensibility measuring method includes measuring, based on a measurement item which corresponds to each of evaluation objects (50), a set of biological information (20), the set each derived from a subject (60) while using each of the evaluation objects (50) on a trial basis, and acquiring an evaluation result (80) for each of the evaluation objects (50), the evaluation result (80) including identification information that enables identification of a rank order in which the evaluation objects (50) are compared with each other, based on the measured set of biological information (20).

## Description

### Technical Field

The present invention relates to a sensibility measuring method and a sensibility measuring system.

### Background Art

Conventionally, a sensibility estimation device that measures the sensibility of a subject is known. Such a sensibility estimation device is disclosed in Japanese Patent Laid-Open No. 2018-187287, for example.

A sensibility estimation device described in Japanese Patent Laid-Open No. 2018-187287 acquires the feature amount of an image visually recognized by a user (subject) in a learning mode. Then, the sensibility estimation device acquires the feature amount of a biosignal integrated from a plurality of biosignals (an electroencephalogram signal, a heartbeat signal, an electrooculogram signal, etc.) acquired when the user visually recognizes the image. Furthermore, the sensibility estimation device acquires sensibility information indicating the type and strength of sensibility by an input from the user. The sensibility estimation device described in Japanese Patent Laid-Open No. 2018-187287 learns the relationship (trained model) between the feature amount of the image, the feature amount of the biosignal, and the sensibility information in the learning mode. Then, the sensibility estimation device estimates the sensibility (sensibility information such as rest and excitement) of the user with respect to a real visual target based on the feature amount of an image acquired by imaging the visual target, biological information measured from the user, and the relationship learned in the learning mode when the user visually recognizes the real visual target in an estimation mode.

### Prior Art

### Patent Document

Patent Document 1: Japanese Patent Laid-Open No. 2018-187287

### Summary of the Invention

### Problems to be Solved by the Invention

Conventionally, when an evaluation object such as a newly developed product is compared by evaluation based on the sensibility of the user (subject), a method for acquiring a subjective evaluation by a questionnaire, an interview, or the like has been conceived. However, in subjective evaluation methods such as a questionnaire and an interview, acquired evaluation results are biased by subject's intentions and preferences, for example. When a device that measures human sensibility, such as the sensibility estimation device described in Japanese Patent Laid-Open No. 2018-187287, is used, sensibility can be measured as objective information based on the measured biological information, but specialized knowledge is required to analyze the measured biological information. Therefore, when a plurality of evaluation objects are compared with each other by evaluation based on sensibility, it is difficult to easily acquire information on sensibility based on objective grounds.

The present invention is intended to solve at least one of the above problems. The present invention aims to provide a sensibility measuring method and a sensibility measuring system capable of easily acquiring evaluations of evaluation objects based on objective grounds when a plurality of evaluation objects are compared with other by evaluation based on human sensibility. Means for Solving the Problems

In order to attain the aforementioned object, a sensibility measuring method according to a first aspect of the present invention includes measuring, based on a measurement item which corresponds to each of evaluation objects, a set of biological information, the set each derived from a subject while using each of the evaluation objects on a trial basis, and acquiring an evaluation result for each of the evaluation objects, the evaluation result including identification information that enables identification of a rank order in which the evaluation objects are compared with each other, based on the measured set of biological information.

A sensibility measuring system according to a second aspect of the present invention includes a biological information measuring unit configured to measure, based on a measurement item which corresponds to each of evaluation objects, a set of biological information, the set each derived from a subject while using each of the evaluation objects on a trial basis, and an evaluation result acquirer configured to acquire an evaluation result for each of the evaluation objects, the evaluation result including identification information that enables identification of a rank order in which the evaluation objects are compared with each other, based on the set of biological information measured by the biological information measuring unit.

### Effect of the Invention

In the sensibility measuring method according to the first aspect and the sensibility measuring system according to the second aspect, the set of biological information is measured from the subject while using each of the evaluation objects on a trial basis based on the measurement item which corresponds to each of the evaluation objects. Accordingly, subjective sensibility felt by actually using the evaluation objects on a trial basis can be represented as objective data. Furthermore, the evaluation result for each of the evaluation objects, including identification information that enables identification of the rank order in which the evaluation objects are compared with each other, is acquired based on the measured set of biological information. Accordingly, the results of comparing the evaluation objects can be easily acquired based on the set of biological information representing sensibility as objective data. Consequently, when the evaluation objects are compared with each other by evaluations based on human sensibility, evaluations of the evaluation objects based on objective grounds can be easily acquired. Furthermore, the set of biological information is measured from the subject while using each of the evaluation objects on a trial basis based on the measurement item which corresponds to each of the evaluation objects, and thus the set of biological information can be measured based on the measurement item which corresponds to each of the evaluation objects, taking into consideration the type of evaluation object, a situation in which each of the evaluation objects is used on a trial basis, a person who uses each of the evaluation objects on a trial basis, etc. Therefore, when the set of biological information is measured from the subject while using each of the evaluation objects on a trial basis, the set of biological information of an appropriate measurement item can be easily measured.

### Brief Description of the Drawings

FIG. 1 is a schematic view for illustrating the configuration of a sensibility measuring system according to a first embodiment.
FIG. 2 is a block diagram for illustrating the sensibility measuring system according to the first embodiment.
FIG. 3 is a diagram for illustrating a display of evaluation results according to the first embodiment.
FIG. 4 is a flowchart for illustrating a sensibility measuring method according to the first embodiment.
FIG. 5 is a block diagram for illustrating the configuration of a sensibility measuring system according to a second embodiment.
FIG. 6 is a diagram for illustrating a display for selecting a combination of measurement items according to the second embodiment.
FIG. 7 is a diagram for illustrating a display of a selected combination of measurement items according to the second embodiment.
FIG. 8 is a block diagram for illustrating the configuration of a sensibility measuring system according to a third embodiment.
FIG. 9 is a diagram for illustrating generation of a trained model according to the third embodiment.
FIG. 10 is a diagram for illustrating a display of evaluation results according to a first modified example of the first to third embodiments.
FIG. 11 is a diagram for illustrating a display of evaluation results according to a second modified example of the first to third embodiments.
FIG. 12 is a diagram for illustrating an evaluation object according to a third modified example of the first to third embodiments.

### Modes for Carrying Out the Invention

Embodiments embodying the present invention are hereinafter described on the basis of the drawings.

### First Embodiment

### Overall Configuration of Sensibility Measuring System

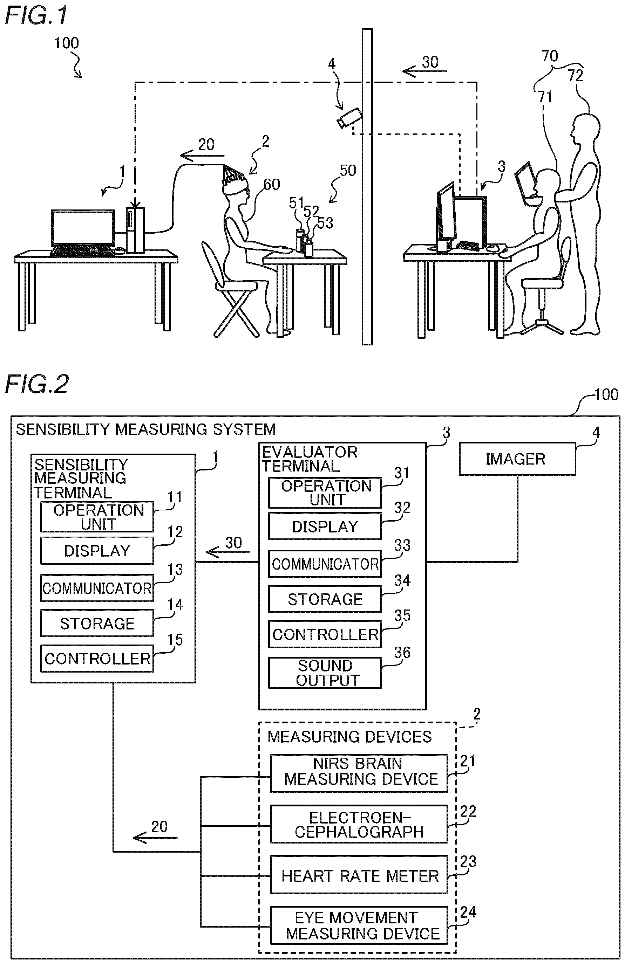

A sensibility measuring system 100 and a sensibility measuring method according to a first embodiment of the present invention are now described with reference to FIGS. 1 to 3.

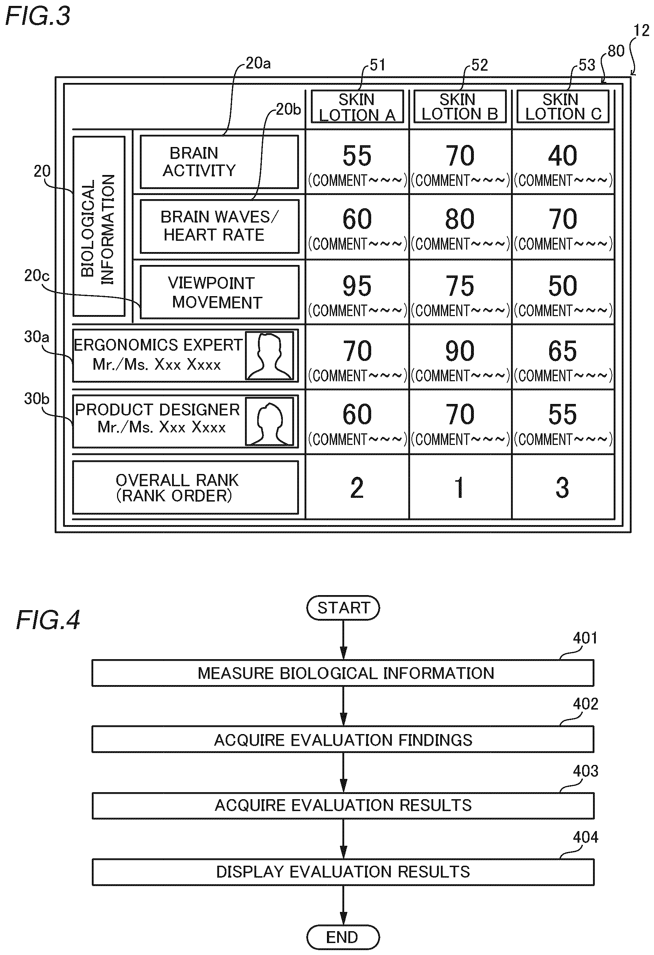

As shown in FIG. 1, the sensibility measuring system 100 acquires evaluation results 80 (see FIG. 3) for a plurality of evaluation objects 50. The plurality of evaluation objects 50 are prototypes made when a company or the like develops a product, for example. The sensibility measuring system 100 objectively measures sensibility felt by a subject 60 when the subject 60 uses the prototype evaluation objects 50 on a trial basis when evaluating the prototype evaluation objects 50 based on sensibility. That is, the sensibility measuring system 100 is used to acquire objective grounds for evaluations that people have sensorily made based on their sensibility when an evaluation object 50 felt preferable is selected from among the plurality of evaluation objects 50.

The evaluation objects 50 are cosmetic products, for example. Specifically, the evaluation objects 50 include a skin lotion 51, a skin lotion 52, and a skin lotion 53. That is, the sensibility measuring system 100 measures how the subject 60 who used each of the skin lotions 51 to 53 on a trial basis has felt about the comfort, smell, appearance of a package, etc. of each of the skin lotions 51 to 53. In other words, the sensibility measuring system 100 evaluates sensibility felt by the subject 60 throughout the experience of "using the skin lotions 51 to 53 on a trial basis". That is, the term "use on a trial basis" indicates not only applying the skin lotions 51 to 53 to the face of the subject 60, but also the entire experience from the time at which the subject 60 picks up containers of the skin lotions 51 to 53 after washing their face to the time at which the subject 60 finishes applying the skin lotions 51 to 53 to their face. The subject 60 is a woman who uses a skin lotion on a daily basis, for example.

As a trial use method (experimental method) suitable for the evaluation objects 50, a method for applying one of the three skin lotions 51 to 53 to the face of the subject 60 at the time at which a predetermined period of time elapses after the subject 60 washes their face in order to make the condition of the skin of the subject 60 uniform is determined, for example. The subject 60 washes their face each time they use one of the three skin lotions 51 to 53 on a trial basis. A preliminary experiment is performed in advance to confirm whether or not the trial use method is suitable for the evaluation objects 50. For example, as the preliminary experiment, a method for using the evaluation objects 50 (skin lotions 51 to 53) on a trial basis at the time at which fifteen minutes elapse after the subject 60 washes their face is determined. Then, it is determined whether or not biological information 20 acquired when the predetermined preliminary experiment is carried out is appropriate. For example, as the preliminary experiment, a method for using the evaluation objects 50 (skin lotions 51 to 53) on a trial basis at the time at which five minutes elapse after the subject 60 washes their face is newly determined when it is confirmed that the evaluation of the evaluation objects 50 is affected due to the increased anxiety and stress of the subject 60 who is concerned about dryness within fifteen minutes after the subject 60 washes their face. When the measurement of sensibility to the evaluation objects 50 is appropriately confirmed in the newly determined preliminary experiment, the contents of the newly determined preliminary experiment are determined to be a trial use method suitable for the evaluation objects 50. The biological information 20 is acquired from the subject 60 while using each of the evaluation objects 50 on a trial basis by the trial use method determined in this manner.

As shown in FIG. 2, the sensibility measuring system 100 includes a sensibility measuring terminal 1, a plurality of measuring devices 2, an evaluator terminal 3, and an imager 4. In the first embodiment, each of the measuring devices 2 measures a set of biological information 20 of the subject 60 while using each of the evaluation objects 50 on a trial basis. The evaluator terminal 3 acquires evaluation findings 30, which are findings of evaluations of the evaluation objects 50 by evaluators 70 (two evaluators 71 and 72). The sensibility measuring terminal 1 acquires the evaluation results 80 (see FIG. 3) for the evaluation objects 50 including the measured set of biological information 20 and the acquired evaluation findings 30. The set of biological information 20, the evaluation findings 30, and the evaluation results 80 are described below in detail. The measuring devices 2 are examples of a "biological information measuring unit" in the claims. The evaluator terminal 3 is an example of an "evaluation finding acquirer" in the claims. The sensibility measuring terminal 1 is an example of an "evaluation result acquirer" in the claims.

### Configuration of Sensibility Measuring Terminal

The sensibility measuring terminal 1 is a computer used to acquire the evaluation results 80 for the evaluation objects 50. The sensibility measuring terminal 1 acquires the set of biological information 20 of the subject 60 measured by each of the measuring devices 2 and acquires the evaluation findings 30 by the evaluators 70 acquired by the evaluator terminal 3. Then, the sensibility measuring terminal 1 acquires the evaluation results 80 based on the acquired set of biological information 20 and the acquired evaluation findings 30. The sensibility measuring terminal 1 includes an operation unit 11, a display 12, a communicator 13, a storage 14, and a controller 15.

The operation unit 11 includes an input device such as a keyboard. The operation unit 11 also includes a pointing device such as a mouse. The operation unit 11 receives an operation signal for operating each portion of the sensibility measuring terminal 1. The operation unit 11 also transmits an operation signal based on an input operation to the controller 15.

The display 12 includes a display monitor such as a liquid crystal display. The display 12 displays the evaluation results 80 for the evaluation objects 50 under the control of the controller 15. An indication on the display 12 is described below in detail.

The communicator 13 can communicate with the measuring devices 2 and the evaluator terminal 3. The communicator 13 transmits and receives information to and from the measuring devices 2 and the evaluator terminal 3. The communicator 13 includes a communication module and an interface for external connection, for example. The communicator 13 communicates by wired or wireless communication. The communicator 13 can communicate with the measuring devices 2 and the evaluator terminal 3 by wired connection via an interface for connecting devices such as a USB cable, an interface for communication such as wired LAN, etc., short-range wireless communication used in wireless LAN, Bluetooth (registered trademark), or an RF tag, or another wireless connection such as infrared communication, for example. The communicator 13 receives the set of biological information 20 from each of the measuring devices 2. The communicator 13 also receives the evaluation findings 30 from the evaluator terminal 3.

The storage 14 is a storage device such as a hard disk drive. The storage 14 stores programs for causing the controller 15 to function. The storage 14 also stores the acquired set of biological information 20 and the acquired evaluation findings 30.

The controller 15 includes a central processing unit (CPU), a read-only memory (ROM), a random access memory (RAM), etc. The CPU executes a predetermined program such that the controller 15 controls each portion of the sensibility measuring terminal 1. Furthermore, the CPU executes a predetermined program such that the controller 15 performs a control to acquire the evaluation results 80. The control by the controller 15 to acquire the evaluation results 80 is described below in detail.

### Measurement of Biological Information

In the first embodiment, each of the measuring devices 2 measures the set of biological information 20 of the subject 60 while using each of the evaluation objects 50 on a trial basis based on each of measurement items which correspond to each of the evaluation objects 50. The measurement items include the type of biological information 20 measured from the subject 60 and a method for measuring the biological information 20. The measurement items are selected according to the type of evaluation objects 50. For example, in the first embodiment, a plurality of (three types of) measurement items including brain activity, brain waves and heartbeats, and viewpoint movement are selected in order to measure sensibility felt by the subject 60 through the experience of applying the skin lotions 51 to 53 to the face. That is, in the first embodiment, the biological information 20 includes the brain blood flow, brain waves, heart rate, and viewpoint movement amount of the subject 60.

Each of the measuring devices 2 measures the set of biological information 20 to measure the sensibility of the subject 60. The measuring devices 2 include a near-infrared spectroscopy (NIRS) brain measuring device 21, an electroencephalograph 22, a heart rate meter 23, and an eye movement measuring device 24.

The NIRS brain measuring device 21 measures the brain blood flow of the subject 60 as the set of biological information 20. Specifically, the NIRS brain measuring device 21 includes a plurality of probes including a light source that emits near-infrared light and a light receiving sensor. The plurality of probes are attached to the head of the subject 60. The NIRS brain measuring device 21 measures of reflected light of the near-infrared light emitted from the light source included in the probes with the light receiving sensor. The NIRS brain measuring device 21 measures the brain blood flow of the subject 60 by measuring hemoglobin in the brain blood of the subject 60 based on the reflected light of the near-infrared light measured by the light receiving sensor.

The electroencephalograph 22 measures the brain waves of the subject 60 as the set of biological information 20. The electroencephalograph 22 includes a plurality of electrodes provided to contact the scalp of the subject 60, and a reference electrode attached to the earlobe or the like of the subject 60. The electroencephalograph 22 measures electrical activity emanating from the brain of the subject 60 by measuring potential differences between the plurality of electrodes and the reference electrode or between the plurality of electrodes.

The heart rate meter 23 measures the heart rate of the subject 60 as the set of biological information 20. The heart rate meter 23 is a wristwatch-type heart rate meter 23 that is wrapped around the wrist of the subject 60. The heart rate meter 23 measures the pulsation of the radial artery inside the wrist of the subject 60.

The eye movement measuring device 24 measures the viewpoint movement amount of the subject 60 as the set of biological information 20. The eye movement measuring device 24 irradiates the eyeballs of the subject 60 with near-infrared light and detects the near-infrared light reflected by the eyeballs of the subject 60 to measure the spatial positions and shapes of the eyeballs of the subject 60. The eye movement measuring device 24 includes a viewpoint camera. The viewpoint camera acquires an image (moving image) of a field of view visually recognized by the subject 60. The eye movement measuring device 24 measures the viewpoint movement amount of the subject 60 based on the measured positions and shapes of the eyeballs of the subject 60 and the image of the field of view of the subject 60 captured by the viewpoint camera.

Each of the plurality of measuring devices 2 transmits the measured set of biological information 20 to the sensibility measuring terminal 1. The measuring devices 2 are wirelessly or wiredly connected to the communicator 13 of the sensibility measuring terminal 1 and can transmit and receive information to and from the sensibility measuring terminal 1. That is, each of the plurality of measuring devices 2 transmits the set of biological information 20 (the brain blood flow, the brain waves, the heart rate, or the viewpoint movement amount) measured from the subject 60 while using each of the skin lotions 51 to 53 on a trial basis to the sensibility measuring terminal 1. One measuring device 2 may measure a plurality of types of biological information 20. For example, the measuring device 2 may be able to measure both the brain waves and the brain blood flow.

### Acquisition of Evaluation Findings

In the first embodiment, the evaluator terminal 3 acquires the evaluation findings 30 obtained by the evaluators 70 selected to correspond to the evaluation objects 50 separately from the subject 60 and evaluating the evaluation objects 50 based on information on the trial use status of each of the plurality of evaluation objects 50 by the subject 60. The evaluators 70 are selected in consideration of the type of evaluation objects 50 and the place and situation in which the evaluation objects 50 are used. That is, the evaluators 70 are selected to correspond to the evaluation objects 50 in comprehensive consideration of how the evaluation objects 50 are used in human behavior, under what circumstances, the evaluation objects 50 are used, and in what time of day or under what situation, the evaluation objects 50 are used, for example.

Specifically, the evaluators 70 include a user experience expert. The term "user experience" refers to impressions and experiences obtained when a product, a system, a service, or the like is used. Specifically, the user experience includes not only the experience of actually using on a trial basis (using) a product, a system, or a service, but also overall experience gained from the use of a product, a system, a service, or the like, including how the product, the system, or the service comes to be used, the influence of using the product, the system, or the service, etc. The evaluators 70 also include an expert who extracts usability problems of the evaluation objects 50 by observing the behavior of the subject 60 who is using each of the evaluation objects 50 on a trial basis. That is, the evaluators 70 include a usability evaluation expert. For example, the two evaluators 70 in the first embodiment are an ergonomics expert evaluator 71 and a product designer evaluator 72.

The evaluators 70 (evaluators 71 and 72) evaluate the evaluation objects 50 by observing the subject 60 who is using each of the evaluation objects 50 on a trial basis. In other words, the evaluators 70 evaluate the evaluation objects 50 being used on a trial basis by the subject 60. Furthermore, the evaluators 70 (evaluators 71 and 72) conduct an interview with or a questionnaire to the subject 60 before and after the trial use of the evaluation objects 50. The evaluators 70 (evaluators 71 and 72) evaluate what kind of influence (experience) has been brought to the subject 60 by using the evaluation objects 50 on a trial basis. In this manner, the evaluators 70 (evaluators 71 and 72) evaluate the evaluation objects 50 based on information regarding the trial use statuses of the evaluation objects 50 by the subject 60.

For example, when evaluating the skin lotions 51 to 53, the evaluators 70 observe the behavior of the subject 60 who is using the skin lotions 51 to 53 on a trial basis, and conduct a questionnaire or the like to the subject 60 before and after the trial use of the skin lotions 51 to 53. Then, the evaluators 70 evaluate the skin lotions 51 to 53 based on information regarding the trial use statuses of the skin lotions 51 to 53 by the subject 60 such as behavior observation and a questionnaire or the like. The evaluators 70 evaluate whether or not there are any portions that the subject 60 finds it difficult to use, and whether or not the shapes and appearances are suitable for the usage situation, for example, based on the information regarding the trial use statuses of the skin lotions 51 to 53 by the subject 60.

The evaluator terminal 3 is a computer used to acquire the evaluation findings 30 that are findings of evaluations of the evaluation objects 50 by the evaluators 70. The evaluation findings 30 include character information indicating the findings of evaluations of the evaluation objects 50 by the evaluators 70. For example, when the evaluation objects 50 are the skin lotions 51 to 53, the evaluation findings 30 are problems of the evaluation objects 50 recognized by the evaluators 70 by observing the behavior of the subject 60, for example. Specifically, the evaluation findings 30 include character information such as that the lid is difficult to open and that the contents are likely to spill out of the container. The evaluation findings 30 also include character information such as that the package design is too childish or too mature given the target age group. The evaluation findings 30 also include evaluation scores of evaluations of the evaluation objects 50 by the evaluators 70. The evaluator terminal 3 includes an operation unit 31, a display 32, a communicator 33, a storage 34, and a controller 35.

The operation unit 31 includes an input device such as a keyboard. The operation unit 31 also includes a pointing device such as a mouse. The operation unit 31 receives input operations by the evaluators 70 for inputting the evaluation findings 30. The operation unit 31 also transmits operation signals based on the input operations to the controller 35.

The display 32 includes a display monitor such as a liquid crystal display. The display 32 displays a moving image of the subject 60 captured by the imager 4 described below. Furthermore, the sound of the subject 60 (such as the voice of the subject 60) who is using the evaluation objects 50 on a trial basis may be output from a sound output 36 such as a speaker.

The communicator 33 can communicate with the sensibility measuring terminal 1 and the imager 4. The communicator 33 transmits and receives information to and from the sensibility measuring terminal 1 and the imager 4. The communicator 33 includes a communication module and an interface for external connection, for example, and communicates by wired or wireless communication. The communicator 33 can communicate with the sensibility measuring terminal 1 and the imager 4 by wired connection via an interface for connecting devices such as a USB cable, an interface for communication such as wired LAN, etc., short-range wireless communication used in wireless LAN, Bluetooth (registered trademark), or an RF tag, or another wireless connection such as infrared communication, for example. The communicator 33 transmits the acquired evaluation findings 30 to the sensibility measuring terminal 1. Furthermore, the communicator 33 receives a video signal from the imager 4.

The storage 34 is a storage device such as a hard disk drive, for example. The storage 34 stores programs for causing the controller 35 to function. The storage 34 also stores the evaluation findings 30.

The controller 35 includes a CPU, a ROM, a RAM, etc. The CPU executes a predetermined control program such that the controller 35 controls each portion of the evaluator terminal 3. The controller 35 causes the display 32 to display an image captured by the imager 4. Furthermore, the controller 35 acquires the evaluation findings 30 including the character information and the evaluation scores based on input operations to the operation unit 31 by the evaluators 70. Then, the controller 35 causes the storage 34 to store the evaluation findings 30 in which the acquired character information and the evaluation scores are associated with each other. The controller 35 transmits the acquired evaluation findings 30 to the sensibility measuring terminal 1 via the communicator 33.

The imager 4 images the subject 60 who is using the evaluation objects 50 on a trial basis. Specifically, the imager 4 images the subject 60 who is using the evaluation objects 50 on a trial basis such that the evaluators 70 can observe the subject 60 who is using the evaluation objects 50 on a trial basis. The imager 4 transmits the captured image (moving image) to the evaluator terminal 3. The imager 4 is installed on a wall surface of an examination room in which the subject 60 uses the evaluation objects 50 on a trial basis. The imager 4 includes an imaging device such as a CMOS image sensor, for example.

Control by Controller of Sensibility Measuring Terminal The controller 15 of the sensibility measuring terminal 1 acquires the set of biological information 20 measured by each of the measuring devices 2 via the communicator 13. Then, the controller 15 performs a process to determine the sensibility of the subject 60 with respect to the acquired set of biological information 20. For example, the controller 15 acquires, based on the set of biological information 20 acquired from each of the measuring devices 2, a numerical value from 1 to 100 representing an evaluation based on the sensibility of the subject 60 as the measurement results of the set of biological information 20.

Specifically, the controller 15 acquires the set of biological information 20 as numerical information so as to correspond to each of the measurement items based on each of the measurement items which correspond to each of the evaluation objects 50. For example, the controller 15 acquires a numerical value of 1 or more and 100 or less indicating the degree of interest of the subject 60 in the evaluation objects 50 (skin lotions 51 to 53) as a set of first biological information 20a based on the brain blood flow (brain activity) acquired by the NIRS brain measuring device 21. A numerical value of 1 or more and 100 or less indicating the degree of comfort of the subject 60 is acquired as a set of second biological information 20b based on the brain waves acquired by the electroencephalograph 22 and the heart rate acquired by the heart rate meter 23. Then, the controller 15 acquires a numerical value of 1 or more and 100 or less indicating the degree of attention of the subject 60 to the evaluation objects 50 as a set of third biological information 20c based on the viewpoint movement amount acquired by the eye movement measuring device 24. The set of biological information 20 as numerical information may be acquired by an input operation to the operation unit 11 based on the set of biological information 20 measured by each of the measuring devices 2. Furthermore, the set of biological information 20 as numerical information may be acquired based on the set of biological information 20 measured by each of the measuring devices 2 by an algorithm such as a trained model generated by machine learning.

The controller 15 acquires a comment corresponding to each of the sets of first to third biological information 20a to 20c as character information based on an input operation to the operation unit 11. For example, the controller 15 acquires a comment such as "the brain activity is increased, and the degree of interest is high" so as to correspond to the set of first biological information 20a. Furthermore, the controller 15 acquires a comment such as "a state in which the sympathetic nerve is dominant has been changed to a state in which the parasympathetic nerve is dominant after the trial use of the skin lotion 51, and thus stress has been relieved by the trial use of the skin lotion 51" so as to correspond to the set of second biological information 20b. Then, the controller 15 causes the storage 14 to store the acquired sets of first to third biological information 20a to 20c and the corresponding comments in association with each other. The comment corresponding to each of the sets of first to third biological information 20a to 20c is acquired based on an input operation to the operation unit 11 by a sensibility measurement expert, for example.

The controller 15 acquires the evaluation findings 30 from the evaluator terminal 3 via the communicator 13. That is, the controller 15 acquires the evaluation findings 30 including character information indicating the findings of evaluations of the evaluation objects 50 by the evaluators 70 and the evaluation scores of the evaluations of the evaluation objects 50 by the evaluators 70. Specifically, the controller 15 acquires evaluation findings 30a of the evaluator 71 who is an ergonomics expert and evaluation findings 30b of the evaluator 72 who is a product designer (see FIG. 3).

In the first embodiment, the controller 15 weights each of the measured plurality of pieces of biological information 20 and the acquired evaluation findings 30. Then, the controller 15 acquires the rank order in which the plurality of evaluation objects 50 are compared with each other based on the weighted biological information 20 and evaluation findings 30. For example, the controller 15 acquires an average value of the measurement results of the plurality of pieces of numerical biological information 20. Then, the controller 15 multiplies the average value of the biological information 20 by 0.6. Furthermore, the controller 15 acquires an average value of the evaluation scores included in the evaluation findings 30 and multiplies the acquired average value of the evaluation scores by 0.4. The controller 15 acquires a total score by adding the average value of the biological information 20 multiplied by 0.6 and the average value of the evaluation scores multiplied by 0.4 for each of the evaluation objects 50. The controller 15 acquires the rank order of the plurality of evaluation objects 50 based on the acquired total score.

As described above, the controller 15 acquires the evaluation results 80 (see FIG. 3) for the evaluation objects 50 that include identification information that enables identification of the rank order in which the plurality of evaluation objects 50 are compared with each other. That is, the controller 15 acquires the evaluation results 80 in which the weighted biological information 20 and evaluation findings 30 are associated with the rank order of the evaluation objects 50 for each of the plurality of evaluation objects 50.

### Indication on Display

As shown in FIG. 3, the controller 15 displays the acquired evaluation results 80 on the display 12. In the first embodiment, the controller 15 arranges and displays the acquired biological information 20 and the evaluation findings 30 for each of the three evaluation objects 50 (skin lotions 51 to 53) on the display 12. The controller 15 displays, on the display 12, the evaluation results 80 including identification information that enables identification of the rank order in which the three evaluation objects 50 (skin lotions 51 to 53) are compared with each other. Specifically, the controller 15 causes the display 12 to display numerical values that are measurement results of three types of biological information 20 (first to third biological information 20a to 20c) corresponding to three types of measurement items and comments corresponding to the biological information 20 (first biological information 20a to third biological information 20c) side by side for each of the three evaluation objects 50 (skin lotions 51 to 53). The controller 15 causes the display 12 to display the evaluation findings 30 (the evaluation findings 30a and 30b including the evaluation scores and the character information) by the two evaluators 70 (evaluators 71 and 72) side by side for each of the three evaluation objects 50 (skin lotions 51 to 53). Then, the controller 15 causes the display 12 to display the rank order in which the three evaluation objects 50 (skin lotions 51 to 53) are compared with each other.

In the first embodiment, the controller 15 displays, on the display 12, the evaluation results 80 with the measured biological information 20 and the acquired evaluation findings 30 displayed in a notation based on a common standard. Specifically, the controller 15 expresses the measurement results of the measured biological information 20 with a numerical value of 1 or more and 100 or less, and expresses the evaluation scores included in the evaluation findings 30 with a numerical value of 1 or more and 100 or less.

### Sensibility Measuring Service

The sensibility measuring system 100 according to the first embodiment is used such that a sensibility measuring service for evaluating the evaluation objects 50 based on sensibility is provided. In this sensibility measuring service, when a customer (such as a company) compares the evaluation objects 50 such as newly developed products by evaluation based on the sensibility of a user (subject), evaluation based on objective grounds is provided. That is, the sensibility measuring service provides the evaluation results 80 for the evaluation objects 50 requested by the customer. Furthermore, in this sensibility measuring service, a place is provided in which the subject 60 is allowed to use the evaluation objects 50 on a trial basis (an experiment is performed) in order to acquire the evaluation results 80 for the evaluation objects 50 using the sensibility measuring system 100. Specifically, a laboratory is provided in which environmental conditions such as humidity, temperature, and illuminance are maintained constant. In the laboratory in which the environmental conditions are maintained constant, each of a plurality of subjects 60 uses the evaluation objects 50 on a trial basis. Thus, even when the plurality of subjects 60 evaluate (use on a trial basis) the plurality of evaluation objects 50 one by one, the conditions for trial use are the same for each of the plurality of evaluation objects 50, and thus it is possible to reduce or prevent the possibility that the biological information 20 measured from the subjects 60 while using the evaluation objects 50 on a trial basis includes variations due to differences in surrounding environmental conditions.

The subjects 60 may be a plurality of (three, for example) women. In this case, the subjects 60 use the evaluation objects 50 on a trial basis one by one. When the subjects 60 are three women, the evaluation results 80 obtained by averaging the biological information 20 and the evaluation findings 30 acquired by the trial use of the evaluation objects 50 by the three women may be acquired, for example. Thus, more detailed evaluation results 80 can be acquired as compared with a case in which the evaluation objects 50 are evaluated by one subject 60. When usability is evaluated, evaluations are performed by the three subjects 60 such that sufficient usability problems and improvements can be extracted, and thus an increase in work time due to evaluations by four or more subjects 60 can be reduced or prevented. The ages of the three women may be restricted according to a request from a company (client).

### Sensibility Measuring Method

A sensibility measuring method using the sensibility measuring system 100 according to the first embodiment is now described with reference to FIG. 4.

First, in step 401, the set of biological information 20 is measured from the subject 60 while using each of the evaluation objects 50 (skin lotions 51 to 53) on a trial basis based on each of the measurement items which correspond to each of the evaluation objects 50. Specifically, the plurality of pieces of biological information 20 (the brain blood flow, the brain waves, the heart rate, and the viewpoint movement amount) are measured so as to correspond to the three types of measurement items (the brain activity, the brain waves and heartbeats, and the viewpoint movement amount) for each of the three skin lotions 51 to 53 based on the three types of measurement items including the type of evaluation objects 50 and the method for measuring the evaluation objects 50.

Then, in step 402, the evaluation findings 30, which are the findings of evaluations of the evaluation objects 50 by the evaluators 70 selected to correspond to the evaluation objects 50 separately from the subject 60, are acquired. Specifically, the evaluation findings 30 including the evaluation scores indicating evaluations by the evaluators 70 (the evaluator 71 who is an ergonomics expert and the evaluator 72 who is a product designer) based on the information on the trial use status by the subject 60 while using each of the evaluation objects 50 on a trial basis, and the character information are acquired based on input operations to the evaluator terminal 3 by the evaluators 70.

Then, in step 403, the evaluation results 80 for the evaluation objects 50 including the measured biological information 20 and the acquired evaluation findings 30 are acquired. Specifically, based on the measured biological information 20, the biological information 20 represented by a numerical value is acquired as the measurement results. Furthermore, the evaluation findings 30 transmitted from the evaluator terminal 3 are acquired. Then, based on the acquired biological information 20 and evaluation findings 30, the evaluation results 80 including identification information that enables identification of the rank order in which the evaluation objects 50 are compared with each other are acquired.

Then, in step 404, the plurality of pieces of biological information 20 and the evaluation findings 30 are displayed side by side, and the evaluation results 80 including identification information that enables identification of the rank order in which the plurality of evaluation objects 50 are compared with each other are displayed. Specifically, for each of the first biological information 20a to the third biological information 20c included in the biological information 20, a numerical value of 1 or more and 100 or less and a comment corresponding to each biological information 20 are displayed side by side on the display 12. Furthermore, for the evaluation findings 30 (evaluation findings 30a and 30b), the evaluation scores and the character information included in the evaluation findings 30 are displayed side by side on the display 12. The rank order in which the plurality of evaluation objects 50 are compared with each other, acquired based on the biological information 20 and the evaluation findings 30, is displayed on the display 12. That is, for each of the plurality of (three) evaluation objects 50 (the skin lotion 51, the skin lotion 52, and the skin lotion 53), the plurality of pieces of biological information 20, the evaluation findings 30, and the corresponding rank order are displayed side by side on the display 12.

Either the process to measure the biological information 20 in step 401 or the process to acquire the evaluation findings 30 in step 402 may be performed first.

### Advantages of Sensibility Measuring Method According to

### First Embodiment

In the sensibility measuring method according to the first embodiment, the following advantages are obtained.

In the sensibility measuring method according to the first embodiment, as described above, the set of biological information 20 is measured from the subject 60 while using each of the evaluation objects 50 on a trial basis based on each of the measurement items which correspond to each of the evaluation objects 50. Accordingly, subjective sensibility felt by actually using the evaluation objects 50 on a trial basis can be represented as objective data. Furthermore, the evaluation results 80 for the evaluation objects 50, including identification information that enables identification of the rank order in which the plurality of evaluation objects 50 are compared with each other, are acquired based on the measured set of biological information 20. Accordingly, the results of comparing the plurality of evaluation objects 50 can be easily acquired based on the biological information 20 representing sensibility as objective data. Consequently, when the plurality of evaluation objects 50 are compared with each other by evaluations based on human sensibility, evaluations of the evaluation objects 50 based on objective grounds can be easily acquired. Furthermore, the set of biological information 20 is measured from the subject 60 while using each of the evaluation objects 50 on a trial basis based on each of the measurement items which correspond to each of the evaluation objects 50, and thus the set of biological information 20 can be measured based on each of the measurement items which correspond to each of the evaluation objects 50, taking into consideration the type of evaluation objects 50, a situation in which the evaluation objects 50 are used on a trial basis, a person who uses the evaluation objects 50 on a trial basis, etc. Therefore, when the set of biological information 20 is measured from the subject 60 while using each of the evaluation objects 50 on a trial basis, the set of biological information 20 of an appropriate measurement item can be easily measured.

In the first embodiment, with the following configurations, further advantages are obtained.

That is, in the first embodiment, as described above, the sensibility measuring method further includes the step of acquiring the evaluation findings 30 obtained by the evaluators 70 selected to correspond to the evaluation objects 50 separately from the subject 60 and evaluating the evaluation objects 50 based on the information on the trial use status of each of the plurality of evaluation objects 50 by the subject 60, and the step of acquiring the evaluation results 80 includes the step of acquiring the evaluation results 80 including the measured set of biological information 20 and the acquired evaluation findings 30. Accordingly, from the objective viewpoint of the evaluators 70 who are different from the subject 60 who actually uses the evaluation objects 50 on a trial basis and have observed the trial use status by the subject 60, information obtained by the behavior observation by the evaluators 70 can be acquired as the evaluation findings 30 of the evaluation objects 50. Therefore, in addition to the information on sensibility represented as data, objective evaluations of the evaluation objects 50 can be acquired from the standpoint of a third party. Consequently, the biological information 20 represented as data about sensibility felt by the subject 60 when the subject 60 actually uses the evaluation objects 50 on a trial basis, and the evaluation findings 30 objectively evaluated from the standpoint of a third party can be acquired, and thus when the evaluation objects 50 are evaluated based on sensibility, evaluations based on objective grounds can be easily acquired.

In the first embodiment, as described above, the step of acquiring the evaluation findings 30 includes the step of acquiring the evaluation findings 30 including the character information indicating the findings of evaluations by the evaluators 70 including an expert on impressions and experiences (user experiences) obtained when a product, a system, or a service is used. Accordingly, the user experience expert's findings can be used to acquire evaluations of user experiences obtained when the subject 60 uses the evaluation objects 50 on a trial basis. Therefore, from the objective viewpoint of the evaluators 70 who have specialized knowledge about user experiences, different from the subjective viewpoint of the subject 60 who is actually using the evaluation objects 50 on a trial basis, evaluations of user experiences provided by the evaluation objects 50 to the subject 60 can be acquired. Consequently, when the evaluation objects 50 are evaluated based on sensibility, more detailed evaluations based on objective grounds based can be acquired based on the specialized knowledge about user experiences.

In the first embodiment, as described above, the step of acquiring the evaluation findings 30 includes the step of acquiring the evaluation findings 30 including the findings of the expert who extracts usability problems of the evaluation objects 50 by observing the behavior of the subject 60 who is using the evaluation objects 50 on a trial basis. Accordingly, the evaluation objects 50 being used by the subject 60 on a trial basis are observed such that regarding an evaluation of a usability problem, such as whether or not each of the evaluation objects 50 has an appearance and shape suitable for the usage environment or whether or not each of the evaluation objects 50 has a point that makes it difficult for the subject 60 to use the evaluation objects 50 on a trial basis, evaluation findings can be acquired from a usability expert different from the subject 60 who is actually using the evaluation objects 50 on a trial basis. Consequently, it is possible to acquire the expert's findings on potential problems of the evaluation objects 50 that the subject 60 who is actually using the evaluation objects 50 on a trial basis cannot recognize, and thus more detailed evaluation results 80 for the evaluation objects 50 can be acquired.

In the first embodiment, as described above, the step of acquiring the evaluation results 80 includes the step of acquiring the evaluation results 80 including the rank order in which the plurality of evaluation objects 50 are compared with each other based on the set of biological information 20 and the evaluation findings 30. Accordingly, when the evaluation object 50 is selected from among the plurality of evaluation objects 50 based on the sensibility measurement, the rank order of the results of comparing the plurality of evaluation objects 50 can be easily recognized. Therefore, the most excellent evaluation object 50 can be easily selected from among the plurality of evaluation objects 50.

In the first embodiment, as described above, the step of measuring the set of biological information 20 includes the step of measuring the plurality of pieces of biological information 20 corresponding to the plurality of measurement items, respectively, based on the plurality of measurement items which correspond to the evaluation objects 50, and the sensibility measuring method further includes the step of displaying the plurality of pieces of biological information 20 and the evaluation findings 30 side by side for each of the plurality of evaluation objects 50, and displaying the evaluation results 80 including identification information that enables identification of the rank order in which the plurality of evaluation objects 50 are compared with each other. Accordingly, the plurality of pieces of biological information 20 and the evaluation findings 30 are displayed side by side, and thus the plurality of pieces of biological information 20 and the evaluation findings 30 acquired for the evaluation objects 50 can be easily visually compared. For example, even when the plurality of pieces of biological information 20 are measured for each of the plurality of evaluation objects 50, the plurality of pieces of biological information 20 and the evaluation findings 30 can be easily visually compared for each of the plurality of evaluation objects 50. Consequently, the plurality of pieces of biological information 20 and the evaluation findings 30 can be easily visually compared for the plurality of evaluation objects 50, and thus the plurality of evaluation objects 50 can be easily compared based on the sensibility measurement.

In the first embodiment, as described above, the step of displaying the evaluation results 80 includes the step of displaying the evaluation results 80 with the measured set of biological information 20 and the acquired evaluation findings 30 displayed in a notation (notation by a numerical value of 1 or more and 100 or less) based on a common standard. Accordingly, the set of biological information 20 and the evaluation findings 30 are displayed in the notation based on a common standard, and thus the set of biological information 20 and the evaluation findings 30 displayed in the notation based on a common standard can be visually compared. Therefore, as compared with a case in which set of the biological information 20 and the evaluation findings 30 are displayed in different notations, the plurality of evaluation objects 50 can be more easily compared with each other based on the sensibility measurement.

In the first embodiment, as described above, the step of measuring the set of biological information 20 includes the step of measuring the plurality of pieces of biological information 20 corresponding to the plurality of measurement items, respectively, based on the plurality of measurement items which correspond to the evaluation objects 50, and the step of acquiring the evaluation results 80 includes the step of acquiring the evaluation results 80 in which each of the measured plurality of pieces of biological information 20 and the acquired evaluation findings 30 have been weighted. Accordingly, the evaluation results 80 in which the measured biological information 20 and the acquired evaluation findings 30 have been weighted can be acquired, and thus the evaluation results 80 in which either the biological information 20 or the evaluation findings 30 have been emphasized can be acquired. Therefore, it is possible to change which of the biological information 20 and the evaluation findings 30 are emphasized depending on the type of evaluation objects 50, and thus the evaluation results 80 in which the weighting has been appropriately changed so as to correspond to the evaluation objects 50 can be acquired. Consequently, more appropriate evaluation results 80 can be easily acquired so as to correspond to the evaluation objects 50.

In the first embodiment, as described above, the step of measuring the set of biological information 20 includes the step of measuring the set of biological information 20 including at least one of the brain blood flow, the brain waves, the heart rate, or the viewpoint movement of the subject 60. Accordingly, based on at least one of the brain blood flow, the brain waves, the heart rate, or the viewpoint movement of the subject 60, the sensibility of the subject 60 to the evaluation objects 50 can be measured. Therefore, the sensibility can be quantitatively measured based on biological signals that the subject 60 themselves cannot perceive, and thus the sensibility of the subject 60 can be measured as objective data. Consequently, at least one of the brain blood flow, the brain waves, the heart rate, or the viewpoint movement of the subject 60 is measured such that evaluations based on objective grounds can be more effectively acquired.

### Advantages of Sensibility Measuring System According to

### First Embodiment

In the sensibility measuring system 100 according to the first embodiment, the following advantages are obtained.

The sensibility measuring system 100 according to the first embodiment is configured as described above such that the set of biological information 20 is measured from the subject 60 while using each of the evaluation objects 50 on a trial basis based on each of the measurement items which correspond to each of the evaluation objects 50. Accordingly, subjective sensibility felt by actually using the evaluation objects 50 on a trial basis can be represented as objective data. Furthermore, the evaluation results 80 for the evaluation objects 50 including identification information that enables identification of the rank order in which the plurality of evaluation objects 50 are compared with each other are acquired based on the measured set of biological information 20. Accordingly, the results of comparing the plurality of evaluation objects 50 can be easily acquired based on the set of biological information 20 representing sensibility as objective data. Consequently, when the plurality of evaluation objects 50 are compared with each other by evaluations based on human sensibility, evaluations of the evaluation objects 50 based on objective grounds can be easily acquired. Furthermore, the set of biological information 20 is measured from the subject 60 while using each of the evaluation objects 50 on a trial basis based on each of the measurement items which correspond to each of the evaluation objects 50, and thus the set of biological information 20 can be measured based on each of the measurement items which correspond to each of the evaluation objects 50, taking into consideration the type of evaluation objects 50, a situation in which the evaluation objects 50 are used on a trial basis, a person who uses the evaluation objects 50 on a trial basis, etc. Therefore, when the set of biological information 20 is measured from the subject 60 while using each of the evaluation objects 50 on a trial basis, the set of biological information 20 of an appropriate measurement item can be easily measured.

In the first embodiment, the sensibility measuring system 100 further includes the evaluation finding acquirer (evaluator terminal 3) configured to acquire the evaluation findings 30 obtained by the evaluators 70 selected to correspond to the evaluation objects 50 separately from the subject 60 and evaluating the evaluation objects 50 based on the information on the trial use status of each of the plurality of evaluation objects 50 by the subject 60, and the evaluation result acquirer (sensibility measuring terminal 1) is configured to acquire the evaluation results 80 including the set of biological information 20 measured by the biological information measuring unit (measuring device 2) and the evaluation findings 30 acquired by the evaluator terminal 3. Accordingly, from the objective viewpoint of the evaluators 70 who are different from the subject 60 who actually uses the evaluation objects 50 on a trial basis and have observed the trial use status by the subject 60, information obtained by the behavior observation by the evaluators 70 can be acquired as the evaluation findings 30 of the evaluation objects 50. Therefore, in addition to the information on sensibility represented as data, objective evaluations of the evaluation objects 50 can be acquired from the standpoint of a third party. Consequently, the biological information 20 represented as data about sensibility felt by the subject 60 when the subject 60 actually uses the evaluation objects 50 on a trial basis, and the evaluation findings 30 objectively evaluated from the standpoint of a third party can be acquired, and thus when the evaluation objects 50 are evaluated based on sensibility, evaluations based on objective grounds can be easily acquired.

In the first embodiment, the sensibility measuring system 100 further includes the display 12 configured to display the evaluation results 80 acquired by the evaluation result acquirer (sensibility measuring terminal 1). Accordingly, the evaluation results 80 can be easily recognized by visually recognizing the evaluation results 80 displayed on the display 12.

### Second Embodiment

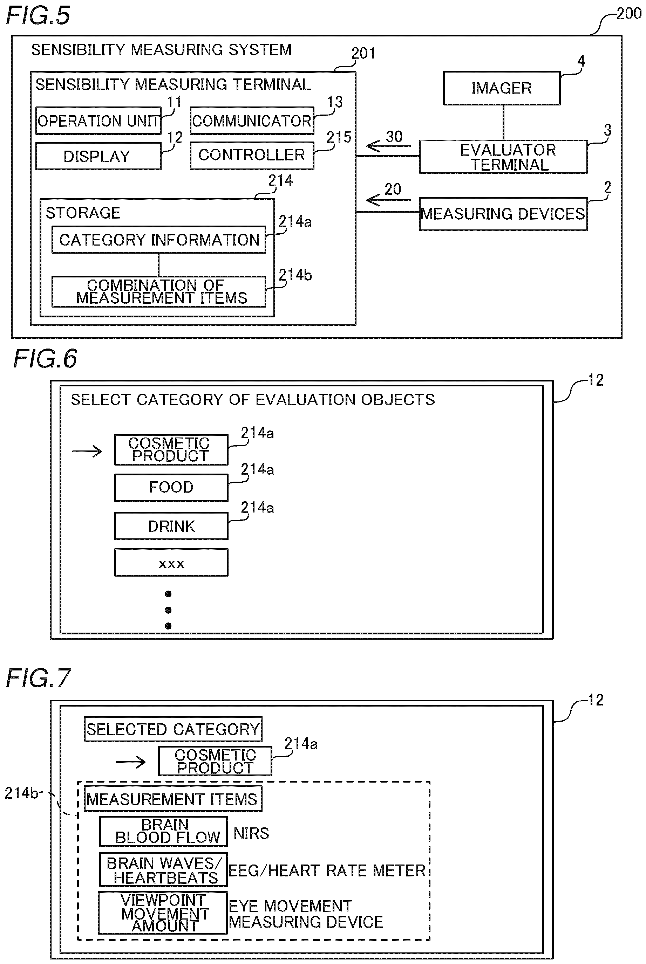

The configuration of a sensibility measuring system 200 according to a second embodiment of the present invention is now described with reference to FIGS. 5 to 7. In this second embodiment, a predetermined combination 214b of measurement items is selected to correspond to evaluation objects 50, unlike the first embodiment. In the figures, the same or similar configurations as those of the first embodiment are denoted by the same reference numerals, and description thereof is omitted.

The sensibility measuring system 200 according to the second embodiment includes a sensibility measuring terminal 201, as shown in FIG. 5. The sensibility measuring system 200 according to the second embodiment acquires evaluation results 80 for the evaluation objects 50 that are skin lotions 51 to 53, similarly to the first embodiment.

The sensibility measuring terminal 201 is a computer used to acquire the evaluation results 80 for the evaluation objects 50, similarly to the sensibility measuring terminal 1 according to the first embodiment. The sensibility measuring terminal 201 includes a storage 214 and a controller 215.

The storage 214 is a storage device such as a hard disk drive, similarly to the storage 14 according to the first embodiment. The storage 214 stores category information 214a indicating the types of evaluation objects 50 and combinations 214b of a plurality of measurement items determined to correspond to the types of evaluation objects 50 in association with each other. The category information 214a includes drink, food, and cosmetic products, for example. The combinations 214b of the measurement items are combinations of measurement items predetermined to correspond to the types of evaluation objects 50 included in the category information 214a. The measurement items are information indicating the types of biological information 20 measured from a subject 60 and measuring devices 2 used to measure the biological information 20, for example. The measurement items may include information defining the characteristics of the subject 60 such as age and sex, a report format for outputting the evaluation results 80, etc.

The controller 215 includes a CPU, a ROM, a RAM, etc., similarly to the controller 15 according to the first embodiment. The CPU executes a predetermined control program such that the controller 215 controls each portion of the sensibility measuring terminal 201. In the second embodiment, the controller 215 selects the predetermined combination 214b of measurement items from among the plurality of measurement items to correspond to the evaluation objects 50. Specifically, the controller 215 selects a combination 214b of measurement items corresponding to a piece of category information 214a selected from among a plurality of pieces of category information 214a stored in the storage 214 based on receiving an input operation to select the piece of category information 214a.

For example, as shown in FIG. 6, the controller 215 causes a display 12 to display the plurality of pieces of category information 214a of the evaluation objects 50 in a selectable manner. Then, the controller 215 acquires an operation signal for selecting the piece of category information 214a from among the plurality of pieces of category information 214a based on the input operation to an operation unit 11. The controller 215 selects the combination 214b of measurement items corresponding to the selected piece of category information 214a based on the acquired operation signal.

As shown in FIG. 7, the controller 215 causes the display 12 to display the selected piece of category information 214a and the combination 214b of measurement items corresponding to the selected piece of category information 214a.

That is, in the second embodiment, the controller 215 selects the predetermined combination 214b of measurement items to correspond to the evaluation objects 50 based on the operation to select the piece of category information 214a from among the plurality of pieces of category information 214a of the evaluation objects 50 stored in advance. Then, the measuring devices 2 acquire the biological information 20 from the subject 60 while using each of the evaluation objects 50 on a trial basis based on the combination 214b of measurement items selected by the controller 215.

The remaining configurations of the second embodiment are similar to those of the first embodiment.

### Advantages of Second Embodiment

In the second embodiment, the following advantages are obtained.

In the second embodiment, as described above, the sensibility measuring method further includes the step of selecting the predetermined combination 214b of measurement items from among the plurality of measurement items to correspond to each of the evaluation objects 50 before the step of measuring the set of biological information 20, and the step of measuring the set of biological information 20 includes the step of measuring a plurality of pieces of biological information 20 corresponding to the measurement items in the selected combination 214b of measurement items based on the selected combination 214b of measurement items. When the evaluation objects 50 are evaluated based on sensibility measurement, it is conceivable to measure the plurality of pieces of biological information 20. For example, it is conceivable to measure many types of biological information 20 such as a blood pressure, an electromyogram of facial muscles, and measurement of a facial expression change by a facial expression sensing device in addition to a brain blood flow, brain waves, a heart rate, and a viewpoint movement amount. Therefore, it is necessary to select the biological information 20 corresponding to the evaluation objects 50 from among such a plurality of types of biological information 20 in order to appropriately evaluate the evaluation objects 50. In consideration of this point, in the second embodiment, the sensibility measuring method further includes the step of selecting the predetermined combination 214b of measurement items from among the plurality of measurement items to correspond to each of the evaluation objects 50 before the step of measuring the set of biological information 20. Accordingly, the predetermined combination 214b of measurement items is selected to correspond to the evaluation objects 50 such that the combination 214b of measurement items corresponding to the evaluation objects 50 can be easily acquired. Therefore, when the sensibility measurement is performed, the combination 214b of measurement items suitable for each type of evaluation objects 50 can be easily acquired, and thus an increase in the time and effort required to select measurement items corresponding to the evaluation objects 50 can be reduced or prevented. The remaining advantages of the second embodiment are similar to those of the first embodiment.

### Third Embodiment

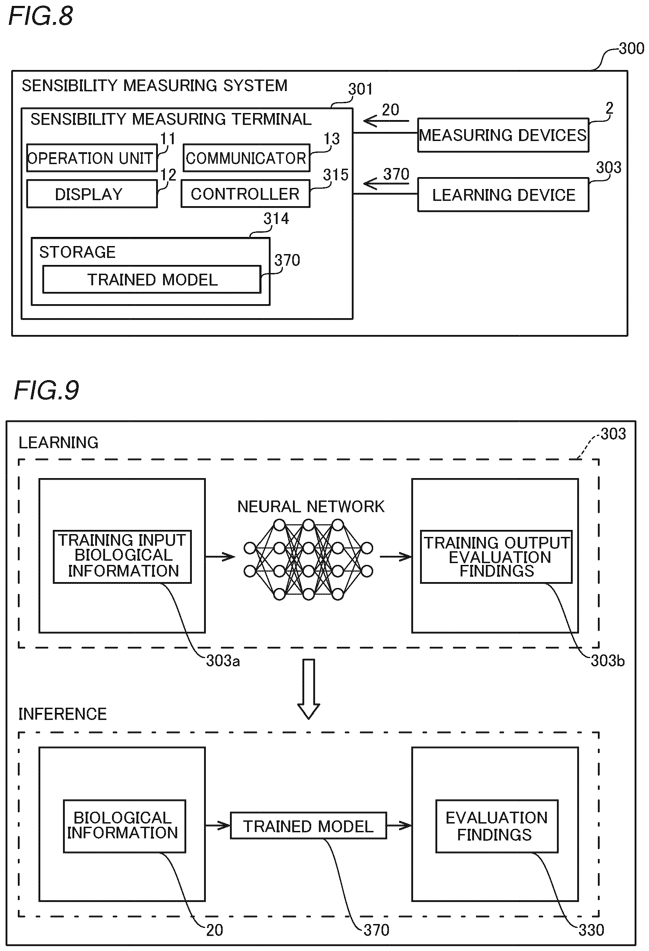

The configuration of a sensibility measuring system 300 according to a third embodiment of the present invention is now described with reference to FIGS. 8 and 9. In the third embodiment, a trained model 370 generated by machine learning is used as evaluators 70, unlike the first embodiment. In the figures, the same or similar configurations as those of the first and second embodiments are denoted by the same reference numerals, and description thereof is omitted.

The sensibility measuring system 300 according to the third embodiment uses the trained model 370 generated by machine learning to acquire evaluation findings 330 including findings of evaluations of evaluation objects 50 output based on measured biological information 20. That is, the sensibility measuring system 300 according to the third embodiment uses the trained model 370 as an evaluator generated by machine learning and selected to correspond to the evaluation objects 50 separately from a subject 60 to acquire the evaluation findings 330.

For example, the sensibility measuring system 300 according to the third embodiment acquires evaluation results 80 for skin lotions 51 to 53 as the evaluation objects 50, similarly to the sensibility measuring system 100 according to the first embodiment. In the sensibility measuring system 300 according to the third embodiment, the evaluation results 80 for the skin lotions 51 to 53 include the biological information 20 measured by measuring devices 2 and the evaluation findings 330 acquired by the trained model 370.

The sensibility measuring system 300 according to the third embodiment includes a sensibility measuring terminal 301 and a learning device 303, as shown in FIG. 8. The sensibility measuring terminal 301 acquires the biological information 20 measured by the measuring devices 2. The sensibility measuring terminal 301 also acquires the evaluation findings 330 using the trained model 370 generated in advance by the learning device 303. The sensibility measuring terminal 301 is an example of an "evaluation finding acquirer" or an "evaluation result acquirer" in the claims.

As shown in FIG. 9, the learning device 303 trains the trained model 370. The trained model 370 is generated by machine learning from training input biological information 303a and training output evaluation findings 303b. Deep learning, which is machine learning using multi-layer neural networks, is used as a machine learning method. The machine learning method is deep learning using fully convolutional networks (FCN), for example. The learning device 303 is a machine learning computer including a CPU, a ROM, a RAM, etc., for example. The learning device 303 can communicate with the sensibility measuring terminal 301 and transmits the generated trained model 370 to the sensibility measuring terminal 301.

For example, when the evaluation results 80 for the skin lotions 51 to 53 are acquired using the trained model 370 generated in advance by the learning device 303, the training input biological information 303a and the training output evaluation findings 303b are acquired using a plurality of types of learning skin lotions different from the skin lotions 51 to 53 in order to train the trained model 370 by the learning device 303. The training input biological information 303a is measured from a learning subject while using each of the learning skin lotions on a trial basis, similarly to a case in which the set of biological information 20 is measured from the subject 60 while using each of the skin lotions 51 to 53 on a trial basis in the first embodiment. The learning subject is a subject different from the subject 60 who tries the skin lotions 51 to 53 on a trial basis. The training output evaluation findings 303b are findings of evaluations of the learning skin lotions acquired by the evaluators 70 by observing the learning subject while using each of the learning skin lotions on a trial basis, similarly to a case in which the evaluation findings 30 are acquired in the first embodiment. The training output evaluation findings 303b are numerical information of 1 or more and 100 or less, for example.

That is, the learning device 303 acquires a plurality of pieces of training input biological information 303a and a plurality of training output evaluation findings 303b corresponding to the training input biological information 303a by performing the same sensibility measurement as in the first embodiment on a plurality of learning subjects using the plurality of types of learning skin lotions. Then, the learning device 303 receives the training input biological information 303a and outputs the training output evaluation findings 303b, performs learning by machine learning, and generates the trained model 370. That is, the learning device 303 trains the trained model 370 by machine learning using the training input biological information 303a and the training output evaluation findings 303b as training data (training set).

The sensibility measuring terminal 301 includes a storage 314 and a controller 315. The sensibility measuring terminal 301 is a computer used to acquire the evaluation results 380 for the evaluation objects 50, similarly to the sensibility measuring terminal 1 according to the first embodiment.

The storage 314 stores the trained model 370 generated by the learning device 303. The storage 314 is a storage device such as a hard disk drive, similarly to the storage 14 according to the first embodiment.

The controller 315 includes a CPU, a ROM, a RAM, etc. The CPU executes a predetermined control program such that the controller 315 controls each portion of the sensibility measuring terminal 301. The controller 315 acquires the trained model 370 generated by the learning device 303 via a communicator 13. Then, the controller 315 causes the storage 314 to store the acquired trained model 370.

The controller 315 acquires the biological information 20 measured by the measuring devices 2. Then, the controller 315 acquires the evaluation findings 330 based on the measured biological information 20 using the trained model 370 stored in the storage 314. The evaluation findings 330 include numerical information of 1 or more and 100 or less, for example.

The remaining configurations of the third embodiment are similar to those of the first embodiment.

### Advantages of Third Embodiment

In the third embodiment, the following advantages are obtained.

In the third embodiment, as described above, the step of acquiring the evaluation findings 330 includes the step of acquiring the evaluation findings 330 including the findings of evaluations of the evaluation objects 50 output based on the measured set of biological information 20 using the trained model 370 generated by machine learning. Accordingly, the trained model 370 can be used to acquire the evaluation findings 330 based on the measured biological information 20, and thus the evaluation findings 330 can be acquired without human evaluators evaluating the evaluation objects 50. Therefore, an increase in the work burden on human evaluators can be reduced or prevented by using the trained model 370 generated by machine learning. The remaining advantages of the third embodiment are similar to those of the first and second embodiments.

### Modified Examples

The embodiments disclosed this time must be considered as illustrative in all points and not restrictive. The scope of the present invention is not shown by the above description of the embodiments but by the scope of claims for patent, and all modifications (modified examples) within the meaning and scope equivalent to the scope of claims for patent are further included.

### First Modified Example

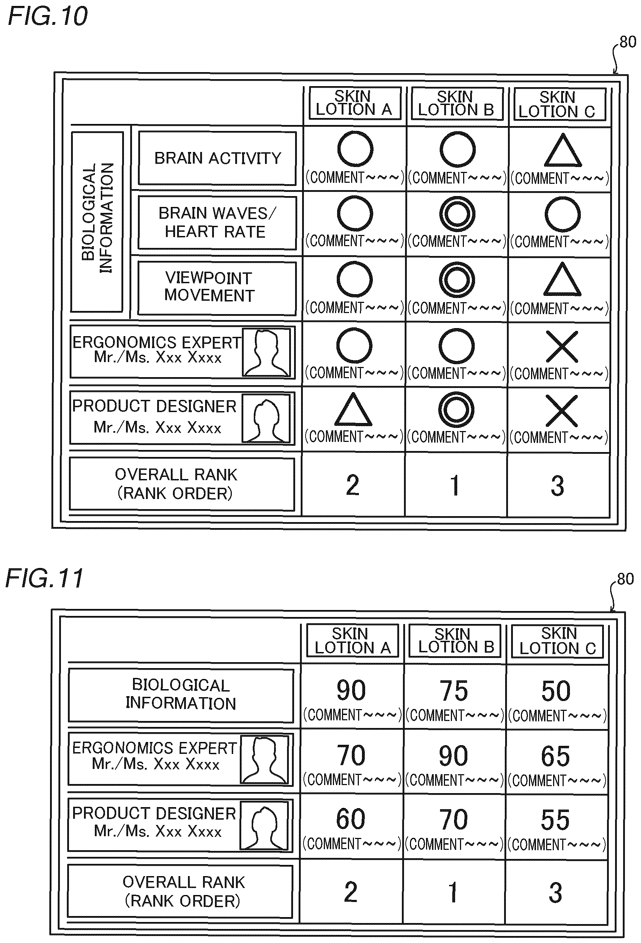

For example, while the example in which the biological information 20 and the evaluation findings 30 represented by numerical values of 1 or more and 100 or less are displayed as the evaluation results 80 on the display 12 has been shown in each of the aforementioned first to third embodiments, the present invention is not limited to this. In the present invention, the biological information and the evaluation results may be represented by symbols indicating four levels of evaluation, like evaluation results 80 according to a first modified example shown in FIG. 10. Specifically, the biological information and the evaluation results may be represented by four symbols of a double circle (a symbol with one circle inside another circle) representing the best evaluation, a single circle (a symbol with one circle) representing the second best evaluation, a triangle representing the third best evaluation, and a cross mark (X mark) representing the worst evaluation.

### Second Modified Example

While the example in which the plurality of pieces of biological information 20 (first to third biological information 20a to 20c) are displayed side by side has been shown in each of the aforementioned first to third embodiments, the present invention is not limited to this. For example, like evaluation results 80 according to a second modified example shown in FIG. 11, one piece of comprehensive biological information generated based on a plurality of pieces of biological information may be displayed. Specifically, measurement results of the plurality of pieces of biological information measured by measuring devices may be represented by numerical values of 1 or more and 100 or less, and an average value of the biological information represented by numerical values of 1 or more and 100 or less may be displayed as one piece of comprehensive biological information on a display (evaluation results).

### Third Modified Example

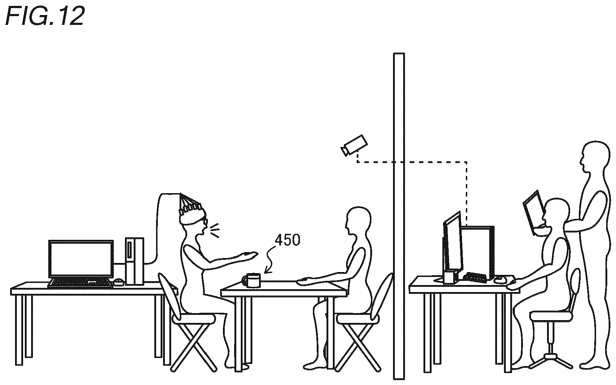

While the example in which the evaluation objects 50 are skin lotions has been shown in each of the aforementioned first to third embodiments, the present invention is not limited to this. For example, like evaluation objects 450 according to a third modified example shown in FIG. 12, the evaluation objects may be coffee. Specifically, when evaluation results for coffee as the evaluation objects are acquired, a concept that "drinking coffee during a meeting activates the discussion" is evaluated. That is, biological information such as brain waves is measured from a subject who is having a discussion while drinking coffee by a measuring device. Then, evaluation findings by evaluators are acquired by observing the subject who is having a discussion while drinking coffee. Then, based on the biological information and the evaluation findings, the evaluation results are acquired in which the degree of concentration, the degree of relaxation, and the activeness of the discussion, for example, have been evaluated.

Furthermore, the biological information 20 measured by the measuring device 2 (biological information measuring unit) may be divided along a time axis and compared. That is, a plurality of pieces of divided biological information 20 may be acquired by dividing the biological information 20 acquired from one type of measuring device 2 along the time axis. Then, the plurality of pieces of acquired biological information 20 may be ranked separately (evaluations based on the sensibility of the subject 60 may be acquired). For example, when coffee is used as the evaluation target 50, like the evaluation object 450 according to the third modified example, the biological information 20 acquired at the time at which the coffee is put in the mouth and the biological information 20 acquired at the time after swallowing of the coffee (aftertaste) may be different from each other. Therefore, an evaluation of the biological information 20 that changes along the time axis can be effectively acquired by dividing the measured biological information 20 along the time axis, as described above. When the biological information 20 is divided along the time axis, it is desirable not to change the weighting for each measuring device 2 (each piece of biological information 20) depending on the time axis. That is, for each of the plurality of pieces of biological information 20 measured by the plurality of measuring devices 2, it is desirable to evaluate the biological information 20 at the time at which the coffee is put in the mouth and the biological information 20 at the time after swallowing of the coffee with common weighting, for example.

### Other Modified Examples

While the example in which the evaluation findings 30 of the evaluation objects 50 by the evaluators 70 are acquired has been shown in each of the aforementioned first and second embodiments, and the example in which the evaluation findings 330 are acquired (inferred) based on the trained model 370 has been shown in the aforementioned third embodiment, the present invention is not limited to this. For example, the evaluation results may be acquired based on the measured biological information without acquiring the evaluation findings.

While the example in which the evaluators 70 include an expert on impressions and experiences (user experiences) obtained when a product, a system, or a service is used has been shown in each of the aforementioned first and second embodiments, the present invention is not limited to this. For example, the evaluators may include an expert in the field corresponding to the evaluation objects. That is, when the evaluation objects are food and drink, the evaluators may include a fragrance expert such as a perfumer.

While the example in which the evaluation findings 30 include the findings obtained by observing the behavior of the subject who is using the evaluation objects 50 on a trial basis has been shown in each of the aforementioned first and second embodiments, the present invention is not limited to this. For example, the evaluation findings may be findings acquired by the evaluators themselves using the evaluation objects on a trial basis.

While the example in which the evaluation findings 30 by the evaluators 70 are acquired by the evaluators 70 observing the subject 60 who is using the evaluation objects 50 on a trial basis while measuring the biological information 20 has been shown in each of the aforementioned first and second embodiments, the present invention is not limited to this. For example, the evaluation findings by the evaluators may be acquired by the evaluators observing the subject who is using the evaluation objects on a trial basis without wearing a device for acquiring the biological information at the timing different from the timing at which the biological information is measured from the subject.

While the example in which the evaluation results 80 including the rank order in which the plurality of evaluation objects 50 are compared with each other are acquired has been shown in each of the aforementioned first to third embodiments, the present invention is not limited to this. For example, the evaluation results including an overall evaluation score for a plurality of evaluation results may be acquired. Alternatively, instead of a rank order, an evaluation object that has obtained the best evaluation among the plurality of evaluation objects may be announced (displayed) in a distinguishable manner. Alternatively, only evaluation results for one of the plurality of evaluation objects may be acquired. Alternatively, questionnaire results may be included in the evaluation results.

While the example in which the evaluation results 80 are displayed with the measured biological information 20 and the acquired evaluation findings 30 displayed in a notation based on a common standard has been shown in each of the aforementioned first to third embodiments, the present invention is not limited to this. For example, the biological information and the evaluation findings may be displayed in notations based on different standards. That is, the biological information may be displayed in a score notation, and the evaluation findings may be displayed on a three-point scale (A, B, and C, for example).

While the example in which the evaluation results 80 in which the measured plurality of pieces of biological information 20 and the acquired evaluation findings 30 have been weighted are acquired has been shown in each of the aforementioned first to third embodiments, the present invention is not limited to this. For example, the evaluation results may be acquired by evenly evaluating the scores of the measured plurality of pieces of biological information and the scores of the evaluation findings without weighting.

While the example in which the biological information 20 includes at least one of the brain blood flow, brain waves, heart rate, or viewpoint movement amount of the subject 60 has been shown in each of the aforementioned first to third embodiments, the present invention is not limited to this. For example, a respiratory rate, a body temperature, the amount of perspiration, a pupil diameter, or the like may be acquired as the biological information. Alternatively, an unconscious behavior of the subject, such as a nodding motion or a facial expression, may be quantified and acquired as the biological information.

While the example in which the sensibility measuring method further includes the step of selecting the predetermined combination of measurement items from among the plurality of measurement items to correspond to each of the evaluation objects 50 has been shown in the aforementioned second embodiment, the present invention is not limited to this. For example, one measurement item may be selected from among the plurality of measurement items to correspond to each of the evaluation objects.

While the example in which the evaluation findings 330 are acquired using the trained model 370 generated by machine learning has been shown in the aforementioned third embodiment, the present invention is not limited to this. For example, instead of machine learning, an algorithm generated by a rule base may be used to acquire the evaluation findings of the evaluation objects.

While the example in which the evaluation results 80 are displayed on the display 12 has been shown in each of the aforementioned first to third embodiments, the present invention is not limited to this. For example, the evaluation results may be output by printing on paper or the like. Alternatively, the acquired evaluation results may be output to the outside via the communicator.

While the example in which the evaluators 70 evaluate the evaluation objects 50 by observing the subject 60 imaged by the imager 4 has been shown in each of the aforementioned first and second embodiments, the present invention is not limited to this. For example, the evaluators may evaluate the evaluation objects by directly observing the subject. In this case, the sensibility measuring terminal may acquire the evaluation findings based on input operations by the evaluators to the operation unit of the sensibility measuring terminal.

While the example in which the evaluators 70 evaluate the evaluation objects 50 by observing the subject 60 displayed on the display 32 of the evaluator terminal 3 by the evaluator terminal 3 acquiring a moving image from the imager 4 has been shown in each of the aforementioned first and second embodiments, the present invention is not limited to this. For example, the moving image captured by the imager may be stored in the storage of the sensibility measuring terminal, and the evaluators may observe the subject who is using the evaluation objects on a trial basis by visually recognizing the stored moving image.

While the example in which the biological information 20 and the evaluation findings 30 are stored in the storage 14 of the sensibility measuring terminal 1 has been shown in each of the aforementioned first to third embodiments, the present invention is not limited to this. For example, the biological information and the evaluation findings may be stored in a database built on a cloud (cloud computing). In this case, the sensibility measuring terminal may acquire the evaluation results based on the biological information and the evaluation findings stored on the cloud.

While the example in which the evaluators 70 are an ergonomics expert and a product designer has been shown in each of the aforementioned first and second embodiments, the present invention is not limited to this. For example, the evaluators may be a human centered design expert. Alternatively, the evaluators may be an ethnographer.

### Aspects

It will be appreciated by those skilled in the art that the exemplary embodiments described above are specific examples of the following aspects.

### (Item 1)

A sensibility measuring method comprising:
measuring, based on a measurement item which corresponds to each of evaluation objects, a set of biological information, the set each derived from a subject while using each of the evaluation objects on a trial basis; and
acquiring an evaluation result for each of the evaluation objects, the evaluation result including identification information that enables identification of a rank order in which the evaluation objects are compared with each other, based on the measured set of biological information.

### (Item 2)

The sensibility measuring method according to item 1, further comprising:
acquiring an evaluation finding obtained by an evaluator selected to correspond to the evaluation objects separately from the subject, the evaluator evaluating the evaluation objects based on information on a trial use status of each of the evaluation objects by the subject; wherein
the acquiring the evaluation result includes acquiring the evaluation result including the measured set of biological information and the acquired evaluation finding.

### (Item 3)

The sensibility measuring method according to item 2, wherein the acquiring the evaluation finding includes acquiring the evaluation finding including character information indicating a finding of an evaluation by the evaluator including an expert on impressions and experiences obtained when a product, a system, or a service is used.

### (Item 4)

The sensibility measuring method according to item 3, wherein the acquiring the evaluation finding includes acquiring the evaluation finding including a finding of the expert who extracts a usability problem of each of the evaluation objects by observing behavior of the subject who is using each of the evaluation objects on a trial basis.

### (Item 5)

The sensibility measuring method according to any one of items 2 to 4, wherein the acquiring the evaluation result includes acquiring the evaluation result including the rank order in which the evaluation objects are compared with each other based on the set of biological information and the evaluation finding.

### (Item 6)

The sensibility measuring method according to any one of items 2 to 5, wherein
the measuring the set of biological information includes measuring, based on a plurality of the measurement items which correspond to the evaluation objects, a plurality of pieces of the biological information corresponding to the plurality of measurement items, respectively; and
the sensibility measuring method further comprises displaying each of the plurality of pieces of biological information and the evaluation finding side by side for each of the evaluation objects, and displaying the evaluation result including the identification information that enables the identification of the rank order in which the evaluation objects are compared with each other.

### (Item 7)

The sensibility measuring method according to item 6, wherein the displaying the evaluation result includes displaying the evaluation result with the measured set of biological information and the acquired evaluation finding displayed in a notation based on a common standard.

### (Item 8)

The sensibility measuring method according to any one of items 2 to 7, wherein
the measuring the set of biological information includes measuring, based on a plurality of the measurement items which correspond to the evaluation objects, a plurality of pieces of the biological information corresponding to the plurality of measurement items, respectively; and
the acquiring the evaluation result includes acquiring the evaluation result in which each of the measured plurality of pieces of biological information and the acquired evaluation finding have been weighted.

### (Item 9)

The sensibility measuring method according to any one of items 1 to 8, wherein the measuring the set of biological information includes measuring the set of biological information including at least one of a brain blood flow, a brain wave, a heart rate, or a viewpoint movement of the subject.

### (Item 10)

The sensibility measuring method according to any one of items 1 to 9, further comprising:
selecting a predetermined combination of measurement items from among a plurality of the measurement items to correspond to each of the evaluation objects before the measuring the set of biological information; wherein
the measuring the set of biological information includes measuring, based on the selected combination of the measurement items, a plurality of pieces of the biological information corresponding to the measurement items in the selected combination of the measurement items, respectively.

### (Item 11)

The sensibility measuring method according to any one of items 2 to 8, wherein the acquiring the evaluation finding includes acquiring the evaluation finding including a finding of an evaluation of each of the evaluation objects output based on the measured set of biological information using a trained model generated by machine learning.

### (Item 12)

A sensibility measuring system comprising:
a biological information measuring unit configured to measure, based on a measurement item which corresponds to each of evaluation objects, a set of biological information, the set each derived from a subject while using each of the evaluation objects on a trial basis; and
an evaluation result acquirer configured to acquire an evaluation result for each of the evaluation objects, the evaluation result including identification information that enables identification of a rank order in which the evaluation objects are compared with each other, based on the set of biological information measured by the biological information measuring unit.

### (Item 13)

The sensibility measuring system according to item 12, further comprising:
an evaluation finding acquirer configured to acquire an evaluation finding obtained by an evaluator selected to correspond to the evaluation objects separately from the subject, the evaluator evaluating the evaluation objects based on information on a trial use status of each of the evaluation objects by the subject; wherein
the evaluation result acquirer is configured to acquire the evaluation result including the set of biological information measured by the biological information measuring unit and the evaluation finding acquired by the evaluation finding acquirer.

### (Item 14)

The sensibility measuring system according to item 12 or 13, further comprising:
a display configured to display the evaluation result acquired by the evaluation result acquirer.

### Description of Reference Numerals

1, 201: sensibility measuring terminal (evaluation result acquirer)
2: measuring device (biological information measuring unit)
3: evaluator terminal (evaluation finding acquirer)
12: display
20: biological information
30, 330: evaluation finding
50, 450: evaluation object
60: subject
70: evaluator
80, 380: evaluation result
100, 200, 300: sensibility measuring system
214b: combination of measurement items
301: sensibility measuring terminal (evaluation finding acquirer, evaluation result acquirer)
370: trained model

## Claims

1. A sensibility measuring method comprising:
measuring, based on a measurement item which corresponds to each of evaluation objects, a set of biological information, the set each derived from a subject while using each of the evaluation objects on a trial basis; and
acquiring an evaluation result for each of the evaluation objects, the evaluation result including identification information that enables identification of a rank order in which the evaluation objects are compared with each other, based on the measured set of biological information.

2. The sensibility measuring method according to claim 1, further comprising:
acquiring an evaluation finding obtained by an evaluator selected to correspond to the evaluation objects separately from the subject, the evaluator evaluating the evaluation objects based on information on a trial use status of each of the evaluation objects by the subject; wherein
the acquiring the evaluation result includes acquiring the evaluation result including the measured set of biological information and the acquired evaluation finding.

3. The sensibility measuring method according to claim 2, wherein the acquiring the evaluation finding includes acquiring the evaluation finding including character information indicating a finding of an evaluation by the evaluator including an expert on impressions and experiences obtained when a product, a system, or a service is used.

4. The sensibility measuring method according to claim 3, wherein the acquiring the evaluation finding includes acquiring the evaluation finding including a finding of the expert who extracts a usability problem of each of the evaluation objects by observing behavior of the subject who is using each of the evaluation objects on a trial basis.

5. The sensibility measuring method according to any one of claims 2 to 4, wherein the acquiring the evaluation result includes acquiring the evaluation result including the rank order in which the evaluation objects are compared with each other based on the set of biological information and the evaluation finding.

6. The sensibility measuring method according to any one of claims 2 to 5, wherein
the measuring the set of biological information includes measuring, based on a plurality of the measurement items which correspond to each of the evaluation objects, a plurality of pieces of the biological information corresponding to the plurality of measurement items, respectively; and
the sensibility measuring method further comprises displaying each of the plurality of pieces of biological information and the evaluation finding side by side for each of the evaluation objects, and displaying the evaluation result including the identification information that enables the identification of the rank order in which the evaluation objects are compared with each other.

7. The sensibility measuring method according to claim 6, wherein the displaying the evaluation result includes displaying the evaluation result with the measured set of biological information and the acquired evaluation finding displayed in a notation based on a common standard.

8. The sensibility measuring method according to any one of claims 2 to 7, wherein
the measuring the set of biological information includes measuring, based on a plurality of the measurement items which correspond to each of the evaluation objects, a plurality of pieces of the biological information corresponding to the plurality of measurement items, respectively; and
the acquiring the evaluation result includes acquiring the evaluation result in which each of the measured plurality of pieces of biological information and the acquired evaluation finding have been weighted.

9. The sensibility measuring method according to any one of claims 1 to 8, wherein the measuring the set of biological information includes measuring the set of biological information including at least one of a brain blood flow, a brain wave, a heart rate, or a viewpoint movement of the subject.

10. The sensibility measuring method according to any one of claims 1 to 9, further comprising:
selecting a predetermined combination of measurement items from among a plurality of the measurement items to correspond to each of the evaluation objects before the measuring the set of biological information; wherein
the measuring the set of biological information includes measuring, based on the selected combination of the measurement items, a plurality of pieces of the biological information corresponding to the measurement items in the selected combination of the measurement items, respectively.

11. The sensibility measuring method according to any one of claims 2 to 8, wherein the acquiring the evaluation finding includes acquiring the evaluation finding including a finding of an evaluation of each of the evaluation objects output based on the measured set of biological information using a trained model generated by machine learning.

12. A sensibility measuring system comprising:
a biological information measuring unit configured to measure, based on a measurement item which corresponds to each of evaluation objects, a set of biological information, the set each derived from a subject while using each of the evaluation objects on a trial basis; and
an evaluation result acquirer configured to acquire an evaluation result for each of the evaluation objects, the evaluation result including identification information that enables identification of a rank order in which the evaluation objects are compared with each other, based on the set of biological information measured by the biological information measuring unit.

13. The sensibility measuring system according to claim 12, further comprising:
an evaluation finding acquirer configured to acquire an evaluation finding obtained by an evaluator selected to correspond to the evaluation objects separately from the subject, the evaluator evaluating the evaluation objects based on information on a trial use status of each of the evaluation objects by the subject; wherein
the evaluation result acquirer is configured to acquire the evaluation result including the set of biological information measured by the biological information measuring unit and the evaluation finding acquired by the evaluation finding acquirer.

14. The sensibility measuring system according to claim 12 or 13, further comprising:
a display configured to display the evaluation result acquired by the evaluation result acquirer.
